# EUROPEAN PATENT APPLICATION

(11) **EP 1 473 667 A2**
(43) Date of publication of application: **03.11.2004**
(21) Application number: 04010110.7
(22) Date of filing: 28.04.2004
(51) Int. Cl.: G06N 3/02

(54) **Portable device for classification of medical data**

(30) Priority: 29.04.2003 GB 0309753
(71) Applicant: Topvine Medical Microsystems Limited, Leighton Buzzard, Bedfordshire LU7 7ZT (GB)
(72) Inventor: Morgan David W, Birmingham, B48 7JX (GB)
(74) Representative: Lockey, Robert Alexander

(57) **Abstract**

A portable device for classification of medical data, the portable device comprising an artificial neural network and a configuration store comprising configuration parameter information relating to the artificial neural network,
the artificial neural network being configured in accordance with configuration parameter information,
the portable device being operable to receive input data,
pass the input data to the ANN, and
receive an output from the ANN.

## Description

### Description of Invention

This invention relates to a portable device for classification of medical data and, a method of providing a portable device and a method of using such a portable device.

An important consideration in the identification of medical conditions is the process of obtaining medical or biomedical data from a patient and the subsequent step of interpreting the acquired data. Problems can arise from both of these initial steps. For example, practical considerations often dictate that medical information can only be obtained from a person at particular times and locations, for example, a person must attend a clinic or hospital to be tested using equipment at that location. Where it is desirable to monitor the person, that is obtain data over an extended period in order for example to identify a change in the condition over an extended period of time or to generate a warning in the event of a sudden change, it is necessary for the person either to remain in one place for an extended period of time, such as at a hospital, or be provided with means for monitoring their own condition using an appropriate data capture device. The first is potentially inconvenient for the patient, whilst in the latter case it may be that the person is not able to interpret the data and thus must be given instructions about what they should do in response to a given change in condition. In the latter case there is also the trouble that there may be a delay in an expert such as a doctor receiving the relevant data and interpreting it.

In connection with the classification and interpretation of medical and biomedical data, it is known to use expert systems such as artificial neural networks (ANN's) and also for the purpose of clinical prediction based on the data. The technique can be used with a wide variety of data types, for example electronic stethoscopes, multi-sensor arrays such as electronic noses and so on. In some circumstances, such as identifying heart or lung problems from sound data, neural networks have been able to outperform human experts in identifying irregularities and problems from the sound signal. The analysis of electrocardiograms using neural networks, or cardiometrics, is another example.

Deployed artificial neural networks systems are generally one of two types. It is known to provide ANN's which are implemented using a dedicated microprocessor, which defines the ANN architecture and knowledge base. Because the ANN is in effect hard-coded, updating or amendment of the ANN or its knowledge base is not possible without substantial addition of or changes to hardware components.

An alternative approach is to provide a ANN on a personal computer such as a notebook or desktop computer. A generic ANN may be used and may be adapted for a broad range of applications by allowing different ANN architectures and data processing algorithms, but by virtue of the cost, size, portability, power consumption and external connectivity of personal computers, it is limited to specific applications where portability or easy monitoring are not required. A further disadvantage compared to the hardware microprocessor configuration is that the resources provided by a personal computer are considerably in excess of that required for artificial neural networks and such a solution is inefficient on a cost basis.

A further obstacle to developing smaller or portable devices is that established ANN architectures such as the multi layer perceptron (MLP) and radial basis function network (RBFN) are computationally relatively heavy and demand corresponding processor and memory overheads.

An aim of the present invention is to provide a new or improved device for classification of medical data which overcomes one or more of the above problems.

According to a first aspect of the invention we provide a portable device for classification of medical data, the portable device comprising an artificial neural network and a configuration store comprising configuration parameter information relating to the artificial neural network, the artificial neural network being configured in accordance with configuration parameter information, the portable device being operable to receive input data, pass the input data to the artificial neural network, and receive an output from the artificial neural network.

The artificial neural network may be a probabilistic neural network and more specifically a constructive probabilistic neural network.

The artificial neural network may comprise an input layer, a pattern layer and a summation layer, wherein the configuration parameter information comprises a first matrix comprising weight information corresponding to the connections between the input layer and the pattern layer, and a second matrix comprising weight information corresponding to the connections between the summation layer and the pattern layer.

The input data may be received from an auxiliary measurement device.

The portable device may be provided with a wireless connection to the auxiliary measurement device whereby the portable device may receive the input data from the auxiliary measurement device.

The portable device may be operable to receive an instruction set corresponding to the auxiliary measurement device to be used to generate the input data.

The portable device may be operable to perform a pre-processing operation on the input data prior to passing the input data to the artificial neural network.

The portable device may be operable to process the information in accordance with an instruction set.

The portable device may be operable to transmit at least one of the input data and the output from the artificial neural network to an external service.

The portable device may be operable to receive configuration parameter information from an external service and store the configuration parameter information in the configuration store.

The external service may comprise a training artificial neural network, the training artificial neural network being trained to classify the input data, the configuration parameter information being established from the training artificial neural network.

The portable device may be operable to receive the information set and the configuration parameter information from the external service.

The portable device may be adaptable to receive an instruction set corresponding to a further auxiliary measurement device and further configuration parameter information to configure the artificial neural network to classify input data from the further auxiliary medical device.

According to a second aspect of the invention we provide a method of providing a portable device for classification of medical data, the portable device comprising an artificial neural network and a configuration store for holding configuration parameter information relating to the artificial neural network, the method comprising the steps of, on a separate device, training an artificial neural net to classify the intended type of input data, extracting the configuration parameter information from the trained artificial neural net, and transmitting the configuration parameter information to the portable device.

The method may comprise the step of providing an instruction set corresponding to an auxiliary measurement device operable to generate the input data and transmitting the instruction set to the portable device.

According to a third aspect of the invention we provide a method of classifying medical data using a portable device comprising an artificial neural network, comprising the steps of receiving input data, passing the input data to the artificial neural network, and receiving an output from the artificial neural network.

The method may comprise the step of transmitting at least one of the output data to an external service.

The method may comprise the step of receiving the input data from an auxiliary measurement device.

The method may comprise the steps of receiving configuration parameter information relating to the artificial neural network and storing the configuration parameter information in a configuration store of the portable device.

The portable device may comprise a portable device according to the first aspect of the invention.

The portable device may be provided in accordance with the second aspect of the invention.

The invention will now be described by way of example only with reference to the accompanying drawings wherein;
Figure 1 is a diagrammatic illustration of a system including a portable device embodying the present invention,
Figure 2 is a diagrammatic illustration of the architecture of a constructive probabilistic neural network,
Figure 3 is a block diagram of a portable device embodying the present invention, and
Figure 4 is a flow diagram of a method embodying the present invention.

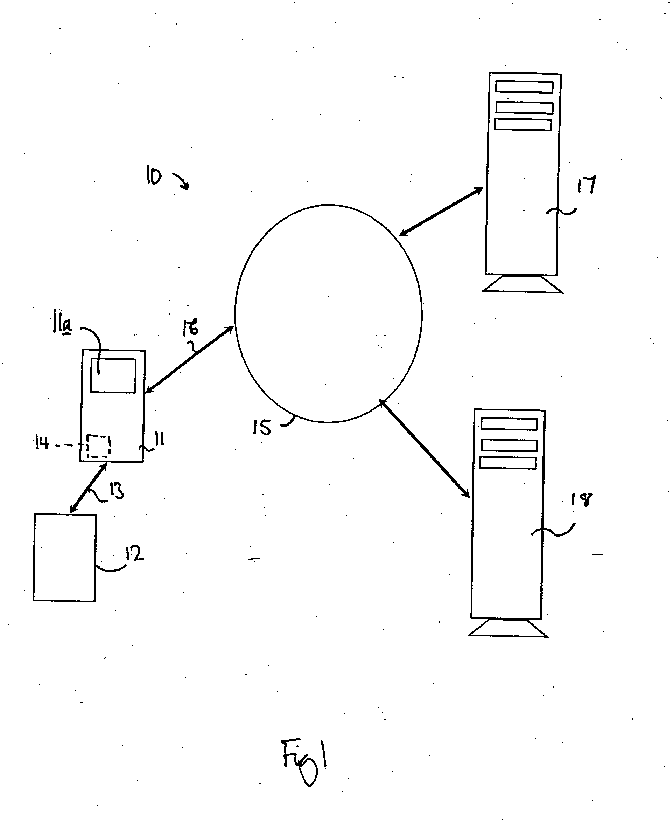

Referring now to Figure 1, a system including a portable device embodying the present invention is generally shown at 10. A portable device embodying the invention is generally shown at 11, in this example operable to communicate with an auxiliary measurement device 12 via a short range communications link 13. The short range communications link 13 may be any appropriate link as desired, for example a physical connection or a wireless connection, such as an infrared link or a radio connection such as a Bluetooth connection. The auxiliary measurement device 12 may be operable to capture any desired medical or biomedical data and transmittal via the communication link 13 to the portable device 11. Alternatively, it might be that the portable device 11 is itself provided with an appropriate measurement device to capture the required data shown in dashed outline at 14, in which case the auxiliary measurement device 12 may be omitted. As an example, where the portable device 11 comprises a mobile phone, the measurement device 12 might simply be the microphone provided as part of the mobile phone. The portable device in this example also has a screen 11a which may be used to provide an output to be viewed by the user.

The portable device 11 is further connected to a communication network generally illustrated at 15, via an appropriate link 16. The network 15 enables the portable device 11 to communicate with any appropriate desired system. In the present example, a training system is generally shown at 17 and an external service operable to receive data is generally shown at 18. The communication network 15 may be any communications network, such as a cellular radio mobile telecommunications network, a public switched telephone network, the Internet or any other communication network or combination of networks as desired. For example, the portable device 11 may communicate with a cellular radio telephone network to connect to a service providing internet access; and so connect to the training service 17 or external data receiving service 18 via the Internet.

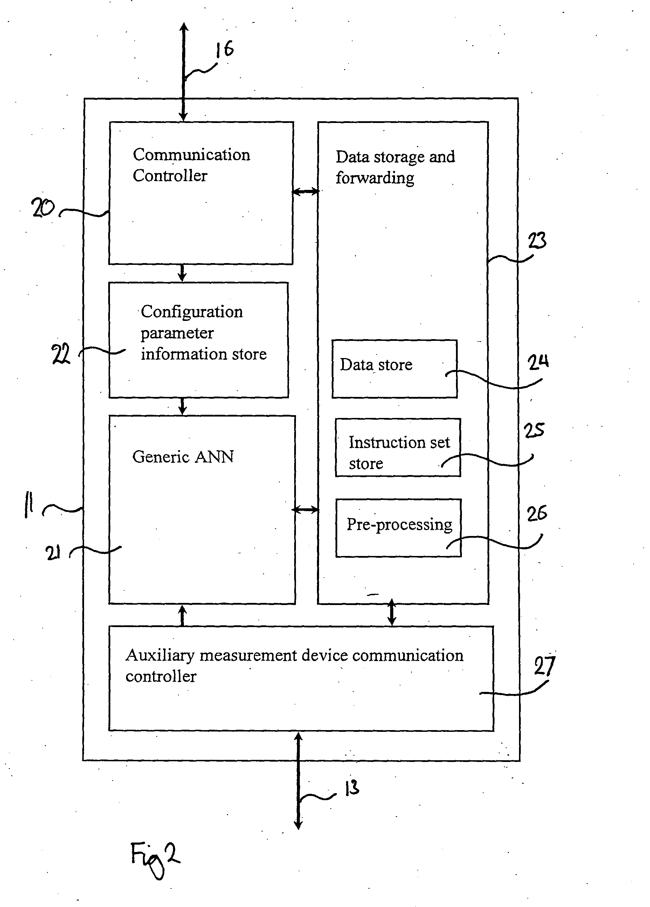

An architecture for the portable device is generally shown in Figure 2. With reference to Figure 2, the portable device 11 comprises a communication controller 20 operable to establish the communication link 16 with the communication network 15. Preferably, this communications link is secure; advantageously, where the portable device 11 comprises a mobile phone, the controller 20 may be operable to establish a digital GSM link with a cellular radio telephone network. To provide for classification of medical data, the portable device is provided with an artificial neural network (ANN). To implement this, a generic artificial neural network is provided as illustrated at 21, configured in accordance with configuration perimeter information held in the configuration parameter store 22. The artificial neural network 21 will be discussed in more detail below.

A data storage and forwarding block is generally shown at 23, which in this example comprises a data store 24 in which the medical or biomedical data may be stored and an instruction set store 25 for holding instructions relating to operation of the auxiliary measurement device 12. The data storage and forwarding block 23 may also be provided with a pre-processor instruction block 26 enabling the portable device 10 to process data before the data is passed to the artificial neural network 21.

An auxiliary measurement device communication controller is shown at 27 operable to link to an auxiliary measurement device 12 to receive data from the auxiliary measurement device 12 and pass it to the artificial neural network 21 and/or the data storage and forwarding block 23, and to transmit control instructions to the auxiliary medical device 12 over the link 13.

In the present example, the mobile device 11 is a device capable of executing applications written for the Symbian (RTM) OS Series 60 using the Java J2ME and C⁺⁺ programming languages, the processor technology having an operating frequency in the range of 1 to 2 MHz and 1 to 5 megabytes of available memory storage. Further, the operating system provides the constraint that the ANN must be implemented using integer arithmetic. This configuration is purely by way of example and it will be apparent that portable device 11 may be programmed and configured on any appropriate platform and using any appropriate technology or programming language as available. This configuration however does illustrate the requirement for the artificial neural network to be computationally lightweight. In the present example, to represent floating point numbers in an environment which may only handle integer arithmetic the floating point value is converted to two values, namely the mantissa and the exponent, where the exponent is selected to render the mantissa as an integer. These two values can then be manipulated to perform pseudo-floating point calculations.

To discuss the artificial neural network 21 provided on the portable device 11 in more detail, in the present example the artificial neural network comprises a probabilistic neural network, and specifically a constructive probabilistic neural network (CPNN). Such neural networks are known, for example from M. Berthold and J. Diamond, *constructive training of probabilistic neural networks,* Neurocomputing 19 (1998) pp167 to 183. Such artificial neural networks have been unexpectedly identified by the inventors as having particular advantages when applied to a portable device 11 as described herein.

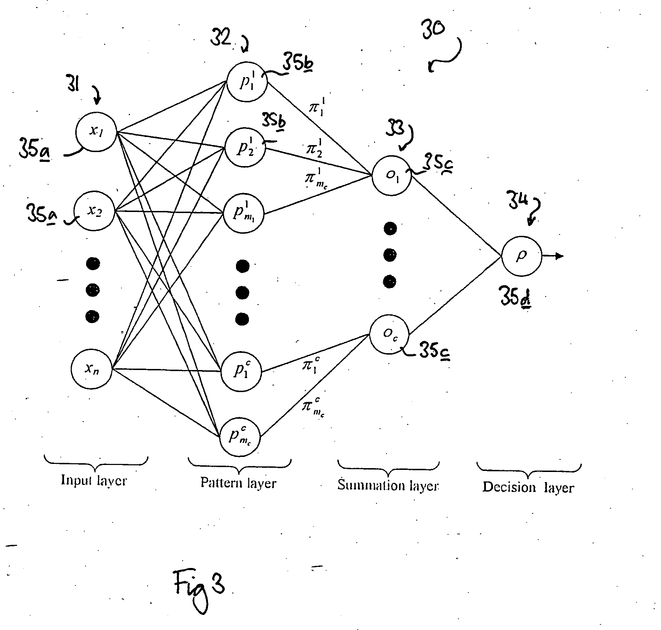

A general architecture for a CPNN is generally shown at 30 in Figure 3. The CPNN has four layers, a first, input layer 31, a second, pattern, layer 32, a third, summation layer 33 and a decision layer shown at 34. Each layer 31, 32, 33, 34 is made up of one or more nodes or neurons 35a, 35b, 35c, 35d respectively. Each neuron 35a in the input layer 31 is connected to each of the neurons 35b of the pattern layer 32 The neuron 35b of the pattern layer 32 are grouped into classes and the output of each neuron 35b of the pattern layer 32 is passed to a neuron 35c in the summation layer 33 corresponding to that class. Finally, the output of each neuron 35c in the summation layer 33 is passed to the neuron 35d of the decision layer 34. In operation, a set of data is supplied to the input layer 31 such that each data point is supplied to one neuron 35a. The input data may be time sequence data, or spectral information or any other appropriate data type as desired. Each neuron 35b of the pattern layer 32 during training is in effect responsive to a particular set of values supplied to the neurons 35a of the input layer 31 and returns an output which, in effect, is dependant on how well the data input to the input layer 31 matches the pattern of data to which the neuron 35b is responsive. The neuron 35c for each class in the summation layer aggregates the output of the neurons 35b in that class by adding up all the outputs modified by a weight given to the output from each neuron 35b. The neuron 35d of the output layer 34 applies a Bayes decision rule to the output of the 35c of the information layer 33 to identify the most likely class in which the input data falls.

The architecture of the CPNN is defined in terms of the number of the neurons in the architecture and the number, configuration and weighting of connections between the layers 31, 32, 33, and can be described using two matrices. The first matrix describes the benefits of the connections, the neuron weight configuration of the pattern layer 32, that is the weight that each node attaches to the input received at each node of the input layer 31. Thus, where the CPNN contains n inputs and a representation of m possible patterns corresponding to m nodes in the pattern layer 32 , the neuron weight will be stored as an n x m matrix. A second matrix describes the connections between the pattern layer 32 and the summation layer 33. Where there are m neurons in layer 32 and p neurons in layer 33, the weights can be stored in an m x p matrix. These weights are reflected in Figure 3 by symbols π¹ ₁ to π^{c} _{mc} as shown in Figure 3. In either matrix, where there is no connection between a pair of neurons at the relevant cell in the matrix the weight is entered as zero. Referring back to figure 2, this is advantageous for the present application in that the portable device 11 may be configured to process any appropriate type of data simply by transmitting the n x m and m x p matrices to the portable device 11 via the communication network 15. The matrices provide the configuration parameter information which is held in the configuration information parameter store 22 and used to provide the architecture for the generic artificial neural network 21. The values in the matrices in the present example contain floating point values, and may be handled in the integer only arithmetic environment by converting them to an integer mantissa and exponent as discussed above.

In the present example, where the ANN comprises a CPNN, a CPNN is preferably trained in an appropriate manner in a separate training system 17. For a CPNN, for example, the training may be performed using a dynamic decay adjustment (DDA algorithm) as discussed in Berthold and Diamond above. Using this algorithm, a pattern is repeatedly input to the input layer 31 of a CPNN network and the parameters and the weights of the neurons and connections are varied until a desired output is generated; in this particular example, the output of one neuron in the correct class has an output greater than a first threshold and that all other neurons in the same class have outputs lower than a second, lower threshold. When this has been achieved for all of the training inputs, the configuration parameter information of the trained CPNN may then be made available, for example via the communication network 15, to any appropriate device having a CPNN such that the devices CPNN may be configured in the same way as the trained CPNN generated by the training service 17.

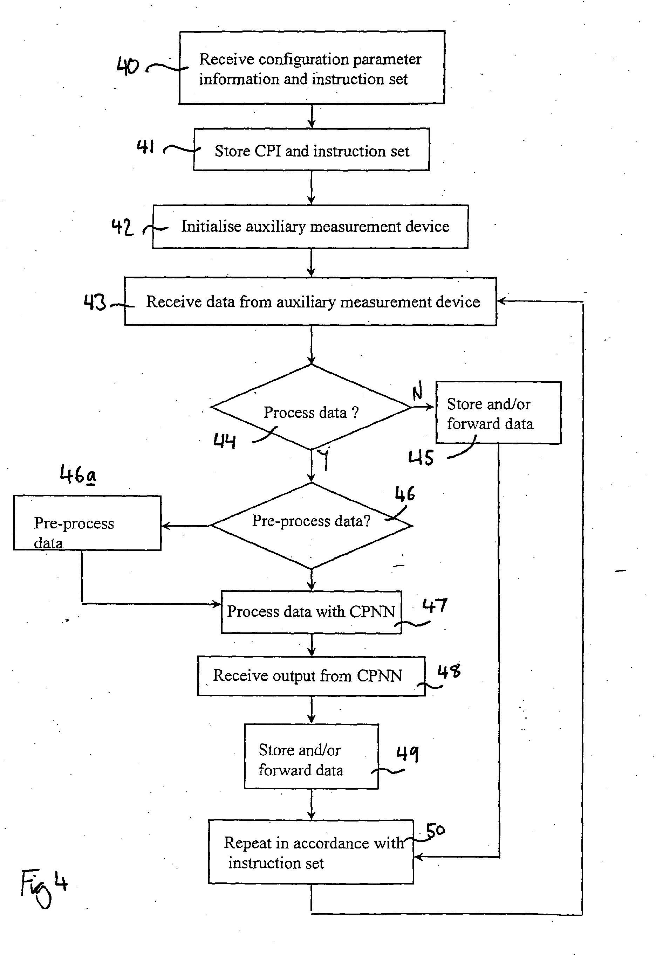

Operation of the portable device 11 will now described by way of example with reference to Figure 4. At step 40, the portable device 11 receives the configuration parameter information and the instruction set for the appropriate medical device 12 for an appropriate service, for example the training service 17. The received configuration parameter information is stored in the appropriate store 22 at step 41 and the instruction set stored in the instruction set store 25. At step 42, the auxiliary medical device may be initialised in accordance with the instruction set as appropriate. At step 43, the relevant data is received from the auxiliary measurement device and at step 44, the portable device 11 checks the instruction set to see whether or not the data should be analysed by the CPNN. If not, at step 45 the data may be stored in the data store 24 and/or transmitted via the communication network 15 to the external service 18. This step may be adapted dynamically by the portable device 11; it may for example by envisaged that the portable communication device is in an area where there is for example no mobile telephone coverage in which case the portable device 11 may store the data in the data store 24 until a communication link 16 is re-established, whereupon the data may be retrieved from the data store 24 and forwarded to the external service 18.

If the data is to be analysed by the CPNN, then at step 46 the portable device 11 will check whether or not the data is to be pre-processed, and if so the pre-processing is performed at step 46a. The pre-processing may be any process as desired, for example conversion from the time domain to frequency domain to generate a spectrum to be processed by the CPNN, or smoothing of data or any other processing step as required.

At step 47, the data is processed by the CPNN which at step 48 generates an output, in this example providing a classification of the input data. At step 49, the output may be stored and/or forwarded to an external service 18, with or without the input data as required. Where the portable device 11 has a screen 11a, a visual display may also be generated instead of or in addition to any other output. At step 50, the portable device 11 checks the instructions and may repeat the process from step 43 in accordance with the instruction set.

The instruction set may include such specific clinical instructions as may be desired. The instructions could include such characteristics, as the monitoring frequency, that is the number of samples to take per-unit time, the analysis frequency, that is the number of analyses using the CPNN which should be performed per unit time, the connectivity frequency, which is the number of connections to the external service 18 that must be established in a given time, instructions on pre-processing the data, such as which data processing algorithms are to be used, and output instructions. The output instructions may be dependent on the output from the CPNN, such that if the CPNN output indicates a particular problem, an alert message may be sent immediately, whereas if the output from the CPNN indicates that a particular parameter is within acceptable bounds, then the data is stored and/or forwarded at the appropriate time in accordance with the connectivity frequency instructions.

It will be apparent that the portable device 11 may be responsive to other instructions, for example instructions sent by the external service 18 via the communication network 15 to, for example, instruct the portable device to generate a status message, or to perform an immediate capture of data, even if the current instructions do not require it to do so at a given time or indeed any appropriate operation as required. The portable device 11 may be notified if there is an updated set of configuration parameter information and/or instructions set for an auxiliary medical device and/or function performed by the portable device 11.

It will be apparent that the present invention provides a highly flexible and adaptable system. The portable device 11 can be reconfigured to monitor any appropriate condition remotely, and may be kept with the patient with no appreciable problem or inconvenience. The portable device 11 may provide both a monitoring service for providing alerts in case of abrupt changes in the person's condition and also provide long term clinical trend data. Possible auxiliary measurement devices may include, but are not limited to; blood pressure monitors; electrocardiograms, pulse monitors, oximeters, respiratory function monitors, electronic stethoscopes, electroencephalograms, plethsmography, ultrasonography, electromyography, electroneuronography, lung and heart sounds, fetal sounds, thermometers, nanotechnology agents and devices, biochemical monitors (e.g. blood sugar), multisensor arrays (e.g. electronic nose).

The input data/output data may be forwarded or acted upon by the external service 18 in any appropriate manner, for example to allow a physician to perform a diagnosis placed on the received data for output, establish the requirements of patients in the context of treatment and/or surgery where the requirements are to be assessed based on the medical data obtained from the active patients, assessing the clerical clinical support required where the long term health of a patient is important, for example, if a patient has a chronic disease, and monitoring a patient's condition after treatment, particularly where data over a long term is required and where monitoring the patient's conditions may be otherwise inconvenient. This is also advantageous given that substantial health organisation resources are devoted to monitoring patients' health.

The portable device 11 may be a mobile telephone or personal digital assistant or any other appropriate device as required.

In the present specification "comprises" means "includes or consists of" and "comprising" means "including or consisting of".

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A portable device for classification of medical data, the portable device comprising an artificial neural network and a configuration store comprising configuration parameter information relating to the artificial neural network,
the artificial neural network being configured in accordance with configuration parameter information,
the portable device being operable to receive input data,
pass the input data to the artificial neural network, and
receive an output from the artificial neural network.

2. A portable device according to claim 1 wherein the artificial neural network is a probabilistic neural network and more specifically a constructive probabilistic neural network.

3. A portable device according to claim 2 wherein the artificial neural network comprises an input layer, a pattern layer and a summation layer, and wherein the configuration parameter information comprises a first matrix comprising weight information corresponding to the connections between the input layer and the pattern layer, and a second matrix comprising weight information corresponding to the connections between the summation layer and the pattern layer.

4. A portable device according to any one of claims 1 to 3 wherein the input data is received from an auxiliary measurement device.

5. A portable device according to claim 4 provided with a wireless connection to the auxiliary measurement device whereby the portable device may receive the input data from the auxiliary measurement device.

6. A portable device according to claim 4 or claim 5 operable to receive an instruction set corresponding to the auxiliary measurement device to be used to generate the input data.

7. A portable device according to any one of the preceding claims where the portable device is operable to perform a pre-processing operation on the input data prior to passing the input data to the artificial neural network.

8. A portable device according to any one of the preceding claims wherein the portable device is operable to process the information in accordance with an instruction set.

9. A portable device according to any one of the preceding claims operable to transmit at least one of the input data and the output from the artificial neural network to an external service.

10. A portable device according to any one of the preceding claims operable to receive configuration parameter information from an external service and store the configuration parameter information in the configuration store.

11. A portable device according to claim 10, wherein the external service comprises a training artificial neural network, the training artificial neural network being trained to classify the input data, the configuration parameter information being established from the training artificial neural network.

12. A portable device according to claim 10 or claim 11 where dependent directly or indirectly on claim 6, wherein the portable device is operable to receive the information set and the configuration parameter information from the external service.

13. A portable device according to any one of claims 10 to 12 which is adaptable to receive an instruction set corresponding to a further auxiliary measurement device and further configuration parameter information to configure the artificial neural network to classify input data from the further auxiliary medical device.

14. A portable device according to any one of the preceding claims wherein the portable device comprises one of a mobile telephone and a personal digital assistant.

15. A method of providing a portable device for classification of medical data, the portable device comprising an artificial neural network and a configuration store for holding configuration parameter information relating to the artificial neural network, the method comprising the steps of,
on a separate device, training an artificial neural net to classify the intended type of input data,
extracting the configuration parameter information from the trained artificial neural net, and
transmitting the configuration parameter information to the portable device.

16. The method of claim 15 further comprising the step of providing an instruction set corresponding to an auxiliary measurement device operable to generate the input data and transmitting the instruction set to the portable device.

17. A method of classifying medical data using a portable device comprising an artificial neural network, comprising the steps of receiving input data, passing the input data to the artificial neural network, and receiving an output from the artificial neural network.

18. A method according to claim 17 comprising the step of transmitting at least one of the output data to an external service.

19. A method according to claim 17 or claim 18 comprising the step of receiving the input data from an auxiliary measurement device.

20. A method according to any one of claims 17 to 19 comprising the steps of receiving configuration parameter information relating to the artificial neural network and storing the configuration parameter information in a configuration store of the portable device.

21. A method according to any one of claims 17 to 20 wherein the portable device comprises a portable device according to any one of claims 1 to 14.

22. A method according to any one of claims 17 to 21 wherein the portable device is provided in accordance with the method of claim 15 or claim 16.
